# DEMANDE DE BREVET EUROPEEN

(11) **EP 1 559 401 A1**
(43) Date de publication de la demande: **03.08.2005**
(21) Numéro de dépôt: 05290142.8
(22) Date de dépôt: 21.01.2005
(51) Int. Cl.: A61K 7/09, A61K 7/135

(54) **Utilisation d'une composition cosmétique obtenue par percolation de vapeur d'eau au travers d'agents réducteurs et/ou oxydants pour le traitement des cheveux**

(30) Priorité: 29.01.2004 FR 0400854
(71) Demandeur: L'OREAL, 75008 Paris (FR)
(72) Inventeur: De la Mettrie, Roland, 78110 Le Vesinet (FR)
(74) Mandataire: Casalonga, Axel

(57) **Abrégé**

L'invention concerne l'utilisation d'une composition obtenue par un procédé comprenant une étape de percolation de vapeur d'eau sous une pression d'au moins 3 bars au travers d'au moins un agent oxydant et/ou d'au moins un agent réducteur, sous forme solide ou pâteuse, pour la décoloration des fibres kératiniques

## Description

La présente invention a pour objet l'utilisation d'une composition cosmétique obtenue par percolation de vapeur d'eau au travers d'agents réducteurs et/ou oxydants pour le traitement des fibres kératiniques, en particulier des fibres kératiniques humaines telles que les cheveux, ainsi qu'un procédé de traitement cosmétique des fibres kératiniques à partir de cette composition.

En cosmétique, pour décolorer des fibres kératiniques, il est usuel d'utiliser des compositions dites oxydantes car elles renferment un ou plusieurs agents aptes à oxyder la mélanine des cheveux et, partant, à la solubiliser pour en obtenir l'élimination totale ou partielle, et des compositions dites, au contraire, réductrices, car elles contiennent un ou plusieurs agents réducteurs comme l'acide ascorbique, les sulfites et les sulfinates, et qui sont plus spécialement destinés à la décoloration des cheveux ayant été antérieurement teints avec des pigments exogènes (décapage).

Par ailleurs, pour la déformation permanente des cheveux, il est usuel d'appliquer sur la chevelure préalablement mise sous tension, par exemple à l'aide de bigoudis si la déformation recherchée est une frisure, une composition contenant un ou plusieurs agents réducteurs de manière à induire l'ouverture des ponts disulfures formés par les résidus cystéine de la kératine des cheveux, puis, généralement après un rinçage, de réoxyder la chevelure pour en fixer la déformation.

Cependant, les compositions de traitement cosmétique contenant de tels agents réducteurs et oxydants sont généralement des compositions aqueuses dans lesquelles lesdits agents doivent être solubilisés. Le manque de solubilité de ces agents diminue l'efficacité de ces compositions. En outre, ce critère de solubilité réduit le nombre d'agents réducteurs et oxydants insolubles dans l'eau que l'on peut utiliser pour le traitement cosmétique des fibres kératiniques. Ceci est particulièrement le cas des composés à point de fusion élevé.

Par ailleurs, ces agents réducteurs sont par essence sensibles à l'oxydation et leur stabilisation en solution aqueuse peut poser des problèmes. Ces problèmes d'instabilité sont encore plus accentués lorsque ces agents réducteurs sont des esters sensibles à l'hydrolyse.

La Demanderesse a découvert de manière surprenante qu'en utilisant un procédé de préparation d'une composition de traitement cosmétique des matières kératiniques, on pouvait obtenir en un temps très court, inférieur à 2 minutes, des compositions plus ou moins concentrées en agents réducteurs et oxydants selon le besoin, notamment sans conservateur, permettant de surmonter les problèmes de solubilité exposés ci-dessus.

Ce procédé est mis en oeuvre simplement. On fait passer un fluide, dont la température est de préférence supérieure ou égale à 30°C, mieux encore allant de 30° à 150°C, et encore plus préférentiellement allant de 40°C à 120°C, sous pression, pendant un laps de temps très court, inférieur à 1 minute, à travers au moins un agent réducteur et/ou au moins un agent oxydant, sous forme solide ou pâteuse, de préférence solide, et encore plus préférentiellement pulvérulente.

Il permet aussi d'utiliser sous forme anhydre des agents réducteurs et/ou oxydants instables dans des compositions aqueuses soit parce qu'ils réagissent avec l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température cosmétiquement acceptable, de préférence inférieure à 60°C. La composition peut être également utilisée jusqu'à une semaine après sa préparation, selon la vitesse de dégradation de l'agent réducteur et/ou oxydant utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les agents réducteurs et/ou oxydants pouvant être conditionnés dans un dispositif prêt-à-l'emploi, il n'est pas nécessaire de déterminer au préalable les concentrations desdits agents en solution, ce qui limite les erreurs de mesure de l'utilisateur.

Le procédé permet d'éviter l'utilisation de flacons multi-compartiments, ce qui rend le procédé particulièrement économique et plus sûr pour l'utilisateur.

En outre, le procédé permet d'associer un agent réducteur et un agent oxydant, ce qui n'est pas possible en solution aqueuse.

La composition ainsi obtenue peut être utilisée seule ou en mélange avec une autre composition.

Un avantage supplémentaire de ce procédé de préparation est l'obtention de compositions conférant de bonnes propriétés cosmétiques. En particulier, les fibres kératiniques traitées avec une composition à base d'agents réducteurs obtenue par le procédé selon l'invention présentent des propriétés de cosmétiques améliorées, et notamment un lissage, une texturisation, ainsi qu'une corporation durables. Les compositions à base d'agents oxydants obtenues par le procédé selon l'invention confèrent aux fibres kératiniques un décapage et une décoloration satisfaisants et durables.

L'invention a donc pour objet l'utilisation d'une composition obtenue par un procédé comprenant une étape de percolation d'un fluide sous une pression d'au moins 3 bars au travers d'au moins un agent oxydant et/ou d'au moins un agent réducteur, sous forme solide ou pâteuse, pour la décoloration des fibres kératiniques.

Elle a également pour objet l'utilisation d'une composition obtenue par un procédé comprenant une étape de percolation d'un fluide sous une pression d'au moins 3 bars au travers d'au moins un agent oxydant et/ou d'au moins un agent réducteur, sous forme solide ou pâteuse, pour la déformation permanente des fibres kératiniques.

L'invention a également pour objet un procédé de décoloration des fibres kératiniques, caractérisé en ce qu'il comprend une étape de préparation d'une composition par percolation d'un fluide sous une pression d'au moins 3 bars au travers d'au moins un agent oxydant et/ou d'au moins un agent réducteur, sous forme solide ou pâteuse, puis l'application de ladite composition sur les fibres kératiniques.

L'invention a également pour objet un procédé de déformation permanente des fibres kératiniques, caractérisé en ce qu'il comprend une étape de préparation d'une composition par percolation d'un fluide sous une pression d'au moins 3 bars au travers d'au moins un agent réducteur et/ou d'au moins un agent oxydant, sous forme solide ou pâteuse, puis l'application de ladite composition sur les fibres kératiniques.

Un autre objet de l'invention est un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Dans ce qui va suivre et à moins d'une indication différente, les bornes délimitant un domaine de valeurs sont comprises dans ce domaine.

Selon l'invention, le procédé de préparation d'une composition de traitement cosmétique des fibres kératiniques comprend une étape de percolation de fluide, de préférence à une température avantageusement supérieure ou égale à 30°C, de préférence allant de 30 à 150°C, mieux encore allant de 40 à 120°C, sous une pression d'au moins 3 bars (3.10⁵ Pa), au travers d'au moins un agent réducteur et/ou d'au moins un agent oxydant, sous forme solide ou pâteuse.

La percolation est un mouvement de fluide à travers un milieu poreux permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, notamment organiques, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par "composé insoluble dans l'eau", on entend tout composé qui à une concentration supérieure ou égale à 0,1% en poids dans l'eau à 25°C, ne forme pas à l'oeil nu une solution isotrope transparente.

L'agent réducteur et/ou l'agent oxydant sont sous forme solide ou pâteuse, de préférence sous forme solide, et encore plus préférentiellement pulvérulente.

On peut utiliser l'agent réducteur seul, l'agent oxydant seul, ou bien les deux ensemble.

Par «forme pâteuse» au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Les fibres kératiniques sont plus particulièrement des fibres kératiniques humaines, comme par exemple les cils, les sourcils et les cheveux.

Le procédé de percolation utilisé dans la présente invention peut être mis en oeuvre à partir d'un dispositif classique permettant de générer un fluide sous pression, à une température de préférence supérieure ou égale à 30°C, de préférence comprise entre 30 et 150°C, mieux encore entre 40 et 120°C. Un tel dispositif comprend une chambre résistant à la pression, équipée d'un bloc thermique, ainsi qu'un circuit d'acheminement du fluide vers l'agent réducteur et/ou oxydant utilisé dans l'invention.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température de préférence supérieure ou égale à 30°C, de préférence comprise entre 30 et 150°C, mieux encore entre 40 et 120°C, sous une pression comprise entre 3 et 30 bars, ou d'au moins 4 bars, de préférence supérieure à 10 bars et tout particulièrement comprise entre 10 et 30 bars.

Le ou les agents réducteurs et oxydants tels que décrits ci-dessous de préférence sous forme solide ou pâteuse, peuvent être utilisés directement dans le dispositif générant le fluide sous pression dans un récipient destiné à cet usage. Ils peuvent aussi être conditionnés dans un dispositif de conditionnement d'une composition cosmétique particulier comprenant un logement fermé délimité par au moins une paroi au moins en partie perméable au fluide sous une pression d'au moins 3 bars, la composition contenant au moins un agent réducteur et/ou un agent oxydant. De tels dispositifs sont, par exemple, décrits dans les demandes de brevets WO 00/56629, EP512470 ou WO 99/003753, US 5897899.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP512470 ou WO 99/003753.

Le ou les agents réducteurs pouvant être utilisés dans le procédé de la présente invention sont notamment choisis parmi ceux généralement utilisés dans le domaine de la décoloration et de la déformation permanente de fibres kératiniques, tels que des sulfites et/ou des bisulfites de métaux alcalins ou alcalino-terreux ou d'ammonium ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en C₁-C₄ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, le dithiodiglycolate de diammonium ou le thioglycolate d'ammonium, et le thioglycérol. On peut également mentionner les composés réducteurs suivants : les N-mercapto-alkylamides de sucres tels que le N-(mercapto-2-éthyl)gluconamide, l'acide β-mercaptopropionique et ses dérivés, l'acide thiomalique, la pantéthéïne, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000 et les alkylaminomercaptoalkylamides décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Les agents réducteurs peuvent également être choisis parmi les réductones tels que l'acide ascorbique, l'acide érythorbique, leurs sels ou leurs esters ainsi que parmi les sulfinates tels que ceux décrits dans les demandes FR2813016 et FR2814948 et en particulier l'acide hydroxyméthanesulfinique et ses sels. Ces réductones et les sulfinates sont particulièrement utilisables dans les procédés de décapage.

L'agent réducteur peut également être un ester sensible à l'hydrolyse.

Les agents réducteurs préférés sont notamment choisis parmi l'acide thioglycolique et ses esters, la cystéamine et la cystéine. L'agent réducteur particulièrement préféré dans l'invention est l'acide thioglycolique et ses esters tels que notamment le monothioglycolate de glycérol ou de glycol, le thioglycolate d'ammonium ou la cystéine.

Le ou les agents oxydants peuvent être choisis parmi les peroxydes d'urée, les bromates tels que les bromates alcalins, les persels et les mélanges de bromates alcalins et d'un persel, les iodates, periodates, chlorates, perchlorates, chlorites, perchlorites, permanganate, notamment de métaux alcalins, et le peroxyde de strontium.

Le ou les agents réducteurs et oxydants de l'invention peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides comme le glucose, le saccharose, le sorbitol, le fructose, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, le ou les agents réducteurs et/ou oxydants de l'invention sont présents de préférence en une quantité allant de 0,5 à 99% en poids, mieux encore de 1 à 80 % en poids, et encore plus préférentiellement de 2 à 60 % en poids par rapport au poids total agent(s) réducteur(s) et/ou oxydant(s) et adjuvant(s).

Les plantes ou extraits de plantes utilisés et au travers desquels passe le fluide sous pression peuvent être soumis avant la percolation à un traitement tel qu'une torréfaction, un cryobroyage, ou une lyophilisation.

La composition de traitement cosmétique des matières kératiniques obtenue selon le procédé de l'invention contient outre le ou les agents réducteur(s) et/ou oxydant(s) et le(s) composant(s) du fluide, à savoir l'eau et/ou le(s) solvant(s) cosmétiquement acceptable(s), éventuellement tout ou partie du ou des adjuvants présents dans le mélange solide ou pâteux.

Les plantes ou extraits de plantes utilisés et au travers desquels passe de la vapeur d'eau sous pression peuvent avoir subi un traitement préalable tel qu'une torréfaction, un cryobroyage ou une lyophilisation.

L'invention concerne également une composition susceptible d'être obtenue par le procédé de percolation, la composition particulièrement préférée étant exempte d'agents conservateurs.

A partir du procédé de préparation de l'invention, on obtient une composition de traitement cosmétique des fibres kératiniques qui peut être appliquée directement sur les fibres kératiniques, ou qui peut être mélangée à un milieu cosmétiquement acceptable, ou encore au moins un additif classiquement utilisé en cosmétique pourra y être ajouté par un opérateur.

On peut également mélanger au moins deux compositions obtenues par le procédé de l'invention.

La composition de traitement cosmétique des fibres kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité du ou des agents réducteurs et/ou oxydants présents dans la composition finale de traitement cosmétique est en général comprise entre 0,001 à 50% en poids environ, de préférence entre 0,005 et 30% en poids, et encore plus préférentiellement entre 0,01 et 20% en poids du poids total de la composition finale de traitement cosmétique.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ, et encore plus préférentiellement entre 5 et 30 % en poids environ par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels adjuvants, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que, par exemple, des huiles de silicone, des agents filmogènes, des agents conservateurs, des agents opacifiants, mais aussi des huiles, des cires, des gommes, des pigments colorés ou nacrés.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale de traitement cosmétique est généralement compris entre 3 et 12 environ, et de préférence entre 5 et 11 environ. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 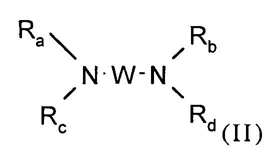 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition finale de traitement cosmétique peut se présenter sous des formes diverses, telles que sous forme de liquides, de crèmes, de mascara, de gels ou sous toute autre forme appropriée pour réaliser un traitement des fibres kératiniques, et notamment des cheveux humains.

La composition finale de traitement cosmétique peut être utilisée, par exemple, comme shampoing, après-shampoing, soin rincé ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

Il est à noter que le procédé de traitement cosmétique des matières kératiniques selon l'invention, peut consister à mettre en oeuvre deux étapes successives.

Ainsi, plus particulièrement dans le cadre d'un procédé de déformation permanente, on peut appliquer une composition comprenant au moins un agent réducteur, obtenue selon le procédé tel que défini ci-dessus, sur les fibres kératiniques, ces dernières étant préalablement mises sous tension (bigoudi ou lissage). Puis, après un temps de pose approprié, on peut appliquer, éventuellement après avoir rincé les fibres, une composition comprenant au moins un agent oxydant, obtenue selon le procédé tel que défini ci-dessus. Après un temps de pose approprié, les fibres sont rincées, lavées avec un shampooing puis rincées à nouveau et enfin séchées ou laissées à sécher.

Les exemples ci-après sont destinés à illustrer la présente invention.

### Exemple 1

On mélange les ingrédients suivants dans les proportions indiquées en % en poids par rapport au poids total de mélange solide :
- L Cystéine commercialisée par la société AJINOMOTO 50 %
- maltodextrine vendue sous la dénomination commerciale 20 % GLUCIDEX 2 par la société ROQUETTE
- bicarbonate d'ammonium commercialisé par la société 30 % RHODIA

On place 5 g de ce mélange dans une machine expresso. On fait ensuite passer de la vapeur d'eau jusqu'à l'obtention d'une composition (A) présentant un volume final de 50 ml.

On peut ensuite ajouter à deux parties en poids de composition (A), une partie en poids d'une composition aqueuse (B) contenant 1 % en poids d'hydroxyéthylcellulose, afin de faciliter l'application.

On obtient ainsi une composition de déformation permanente prête à être appliquée sur les cheveux.

On obtient des cheveux déformés de manière durable, et présentant une corporisation et une texturisation durables.

### Exemple 2

On mélange les ingrédients suivants dans les proportions indiquées en % en poids par rapport au poids total de mélange solide :
- persulfate de potassium 20 %
- chlorure d'ammonium 30 %
- bicarbonate d'ammonium commercialisé par la société 10 % RHODIA
- gomme de guar commercialisés sous le dénomination 40 % GUARGEL D/15 par la société RHODIA

On place 5 g de ce mélange dans une machine expresso. On fait ensuite passer de la vapeur d'eau jusqu'à l'obtention d'une composition (A) présentant un volume final de 50 ml.

On peut ensuite ajouter à deux parties en poids de composition (A), une partie en poids d'une composition aqueuse (B) contenant 1 % en poids d'hydroxyéthylcellulose, afin de faciliter l'application.

On obtient ainsi une composition de décoloration prête à être appliquée sur les cheveux.

### Exemple 3

On mélange les ingrédients suivants dans les proportions indiquées en % en poids par rapport au poids total de mélange solide :
- peroxyde de carbamide commercialisé par la société 10 % PEROXID CHEMIE
- acide érythorbique commercialisé par la société PFIZER 10 %
- bicarbonate d'ammonium commercialisé par la société 60 % RHODIA
- maltodextrine commercialisée sous le dénomination 20 % GLUCIDEX 2 par la société ROQUETTE

On place 5 g de ce mélange dans une machine expresso. On fait ensuite passer de la vapeur d'eau jusqu'à l'obtention d'une composition (A) présentant un volume final de 50 ml.

On peut ensuite ajouter à deux parties en poids de composition (A), une partie en poids d'une composition aqueuse (B) contenant 1 % en poids d'hydroxyéthylcellulose, afin de faciliter l'application.

On obtient ainsi une composition de décoloration prête à être appliquée sur les cheveux.

## Revendications

1. Utilisation d'une composition obtenue par un procédé comprenant une étape de percolation de vapeur d'eau sous une pression d'au moins 3 bars au travers d'au moins un agent oxydant et/ou d'au moins un agent réducteur, sous forme solide ou pâteuse, pour la décoloration des fibres kératiniques.

2. Utilisation d'une composition obtenue par un procédé comprenant une étape de percolation de vapeur d'eau sous une pression d'au moins 3 bars au travers d'au moins un agent réducteur et/ou d'au moins un agent oxydant, sous forme solide ou pâteuse, pour la déformation permanente des fibres kératiniques.

3. Utilisation selon la revendication 1 ou 2, **caractérisée en ce que** le ou les agents réducteurs sont choisis parmi les sulfites et/ou les bisulfites de métaux alcalins ou alcalino-terreux ou d'ammonium, les thiols, les N-mercapto-alkylamides de sucres, l'acide β-mercaptopro-pionique et ses dérivés, l'acide thiomalique, la panthétéïne.

4. Utilisation selon la revendication 3, **caractérisée en ce que** les thiols sont choisis parmi la cystéïne et ses divers dérivés, la cystéamine et ses divers dérivés, l'acide thiolactique et ses esters, l'acide thioglycolique ainsi que ses esters, et le thioglycérol.

5. Utilisation selon la revendication 3, **caractérisée en ce que** l'agent réducteur est choisi parmi l'acide thioglycolique et ses esters, la cystéamine et la cystéine.

6. Utilisation selon la revendication 5, **caractérisée en ce que** l'agent réducteur est choisi parmi le monothioglycolate de glycérol ou de glycol, le thioglycolate d'ammonium et la cystéine.

7. Utilisation selon la revendication 1, **caractérisée en ce que** l'agent réducteur est un ester sensible à l'hydrolyse.

8. Utilisation selon la revendication 1 ou 2, **caractérisée en ce que** l'agent oxydant est choisi parmi les peroxydes d'urée, les bromates, les persels et les mélanges de bromates alcalins et d'un persel, les iodates, periodates, chlorates, perchlorates, chlorites, perchlorites, permanganate, notamment de métaux alcalins, et le peroxyde de strontium.

9. Utilisation selon l'une quelconque des revendications précédentes, **caractérisée en ce que** l'agent oxydant et/ou l'agent réducteur est mis en oeuvre en mélange avec au moins un adjuvant.

10. Utilisation selon la revendication 9, **caractérisée en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, et les particules de poly(chlorure de vinylidène/acrylonitrile).

11. Utilisation selon la revendication 9 ou 10, **caractérisée en ce que** l'agent réducteur et/ou l'agent oxydant sont présents en une quantité allant de 0,5 à 99% en poids, de préférence de 1 à 80 % en poids par rapport au poids total agent réducteur et/ou agent oxydant et adjuvant(s).

12. Utilisation selon l'une quelconque des revendications précédentes, **caractérisée en ce que** l'étape de percolation est mise en oeuvre avec un fluide sous pression compris entre 3 et 30 bars, de préférence entre 10 et 30 bars.

13. Utilisation selon l'une quelconque des revendications précédentes, **caractérisée en ce que** l'étape de percolation est mise en oeuvre avec un fluide sous pression d'au moins 10 bars.

14. Utilisation selon l'une quelconque des revendications précédentes, **caractérisée en ce que** le fluide est de la vapeur d'eau, éventuellement accompagnée d'eau liquide.

15. Utilisation selon l'une quelconque des revendications 1 à 13, **caractérisée en ce que** le fluide est constitué par un ou plusieurs solvants organiques liquides et/ou gazeux cosmétiquement acceptables.

16. Composition de traitement cosmétique des matières kératiniques susceptible d'être obtenue par le procédé défini dans l'une quelconque des revendications 1 à 15.

17. Procédé de décoloration des fibres kératiniques, **caractérisé en ce qu'**il comprend une étape de préparation d'une composition par percolation de vapeur d'eau sous une pression d'au moins 3 bars au travers d'au moins un agent oxydant et/ou d'au moins un agent réducteur, sous forme solide ou pâteuse, puis l'application de ladite composition sur les fibres kératiniques.

18. Procédé de déformation permanente des fibres kératiniques, **caractérisé en ce qu'**il comprend une étape de préparation d'une composition par percolation de vapeur d'eau sous une pression d'au moins 3 bars au travers d'au moins un agent réducteur et/ou d'au moins un agent oxydant, sous forme solide ou pâteuse, puis l'application de ladite composition sur les fibres kératiniques.

19. Procédé de traitement cosmétique des matières kératiniques selon la revendication 17 ou 18, **caractérisé en ce que** l'on applique ladite composition sur les fibres kératiniques par l'intermédiaire d'un dispositif ne nécessitant pas d'intervention humaine.

20. Procédé de traitement cosmétique des matières kératiniques selon l'une quelconque des revendications 17 à 19, **caractérisé en ce que** le ou les agents réducteurs sont choisis parmi les sulfites et/ou les bisulfites de métaux alcalins ou alcalino-terreux ou d'ammonium, les thiols, les N-mercapto-alkylamides de sucres, l'acide β-mercaptopropionique et ses dérivés, l'acide thiomalique, la panthétéïne.

21. Procédé de traitement cosmétique des matières kératiniques selon la revendication 20, **caractérisé en ce que** les thiols sont choisis parmi la cystéïne et ses divers dérivés, la cystéamine et ses divers dérivés, l'acide thiolactique et ses esters, l'acide thioglycolique ainsi que ses esters, et le thioglycérol.

22. Procédé de traitement cosmétique des matières kératiniques selon la revendication 20, **caractérisé en ce que** l'agent réducteur est choisi parmi l'acide thioglycolique et ses esters, la cystéamine et la cystéine.

23. Procédé de traitement cosmétique des matières kératiniques selon l'une quelconque des revendications 18 à 20, **caractérisé en ce que** l'agent réducteur est un ester sensible à l'hydrolyse.

24. Procédé de traitement cosmétique des matières kératiniques selon la revendication 17, **caractérisé en ce que** l'agent oxydant est choisi parmi les peroxydes d'urée, les bromates, les persels et les mélanges de bromates alcalins et d'un persel, les iodates, periodates, chlorates, perchlorates, chlorites, perchlorites, permanganate, notamment de métaux alcalins, et le peroxyde de strontium.

25. Procédé de traitement cosmétique des matières kératiniques selon l'une quelconque des revendications 17 à 24, **caractérisé en ce que** l'agent oxydant et/ou l'agent réducteur est mis en oeuvre en mélange avec au moins un adjuvant.

26. Procédé de traitement cosmétique des matières kératiniques selon la revendication 25, **caractérisé en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, et les particules de poly(chlorure de vinylidène/acrylonitrile).

27. Procédé de traitement cosmétique des matières kératiniques selon la revendication 25 ou 27, **caractérisé en ce que** l'agent réducteur et/ou l'agent oxydant sont présents en une quantité allant de 0,5 à 99% en poids, de préférence de 1 à 80 % en poids par rapport au poids total agent réducteur et/ou agent oxydant et adjuvant(s).

28. Procédé de traitement cosmétique des matières kératiniques selon l'une quelconque des revendications 17 à 27, **caractérisé en ce que** l'étape de percolation est mise en oeuvre avec un fluide sous pression d'au moins 10 bars.

29. Procédé de traitement cosmétique des matières kératiniques selon l'une quelconque des revendications 17 à 28, **caractérisé en ce que** le fluide est de la vapeur d'eau, éventuellement accompagnée d'eau liquide.

30. Procédé de traitement cosmétique des matières kératiniques selon l'une quelconque des revendications 17 à 28, **caractérisé en ce que** le fluide est constitué par un ou plusieurs solvants organiques liquides et/ou gazeux cosmétiquement acceptables.

31. Procédé de traitement cosmétique des matières kératiniques selon l'une quelconque des revendications 17 à 30, **caractérisé en ce que**, avant l'application, la composition de traitement cosmétique préparée selon le procédé est mélangée à un milieu cosmétiquement acceptable et/ou à un ou plusieurs additifs utilisés en cosmétique.

32. Dispositif de conditionnement d'une composition cosmétique, comprenant un logement fermé délimité par au moins une paroi au moins en partie perméable à un fluide sous pression d'au moins 3 bars, le logement contenant au moins un agent réducteur et/ou au moins un agent oxydant sous forme solide ou pâteuse.

33. Dispositif selon la revendication 32, dans lequel le logement est délimité par deux feuilles scellées.

34. Dispositif selon la revendication 32, **caractérisé en ce que** le logement est délimité par une barquette fermée par un couvercle.
